(19) Europäisches Patentamt
European Patent Office
Office européen des brevets

(11) **EP 3 167 056 B1**

(12) **EUROPEAN PATENT SPECIFICATION**

(45) Date of publication and mention
of the grant of the patent:
**11.09.2024 Bulletin 2024/37**

(21) Application number: **15736831.7**

(22) Date of filing: **10.07.2015**

(51) International Patent Classification (IPC):
*C12N 9/64* $^{(2006.01)}$    *A23C 19/032* $^{(2006.01)}$
*C12N 9/08* $^{(2006.01)}$    *C12N 9/96* $^{(2006.01)}$

(52) Cooperative Patent Classification (CPC):
**A23C 19/0326; A23C 19/032; C12N 9/0065;**
**C12N 9/6478; C12N 9/6481; C12N 9/6483;**
**C12N 9/96; C12Y 111/01007; C12Y 304/23001;**
**C12Y 304/23002; C12Y 304/23004;**
**C12Y 304/23023**

(86) International application number:
**PCT/EP2015/065885**

(87) International publication number:
**WO 2016/005587 (14.01.2016 Gazette 2016/02)**

(54) **CAMEL CHYMOSIN ENZYME COMPOSITION WITH IMPROVED PHYSICAL STABILITY**

KAMEL-CHYMOSIN-ENZYMZUSAMMENSETZUNG MIT VERBESSERTER PHYSIKALISCHER STABILITÄT

COMPOSITION ENZYMATIQUE CAMEL CHYMOSINE PRÉSENTANT UNE STABILITÉ PHYSIQUE AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.07.2014 EP 14176744**

(43) Date of publication of application:
**17.05.2017 Bulletin 2017/20**

(73) Proprietor: **Chr. Hansen A/S**
**2970 Hoersholm (DK)**

(72) Inventors:
• **LUND, Martin**
  **2100 Copenhagen Ø (DK)**
• **HANSEN, Enikoe Fodor**
  **3000 Helsingoer (DK)**
• **JACOBSEN, Jonas**
  **2100 Copenhagen Ø (DK)**
• **VAN DEN BRINK, Johannes Maarten**
  **2730 Herlev (DK)**

(56) References cited:
WO-A1-2012/127005    WO-A1-96/19582
WO-A2-95/29999       CN-A- 101 709 295

Remarks:
The complete document including Reference Table(s) and the Sequence Listing(s) can be downloaded from the EPO website

**Description**

**FIELD OF THE INVENTION**

The present invention relates to liquid milk clotting aspartic protease enzyme composition comprising added polypeptides/proteins.

**BACKGROUND ART**

Enzymatic coagulation of milk by milk-clotting enzymes, such as chymosin and pepsin, is one of the most important processes in the manufacture of cheeses. Enzymatic milk coagulation is a two-phase process: a first phase where a proteolytic enzyme, chymosin or pepsin, attacks κ-casein, resulting in a metastable state of the casein micelle structure and a second phase, where the milk subsequently coagulates and forms a coagulum.

Chymosin (EC 3.4.23.4) and pepsin (EC 3.4.23.1), the milk clotting enzymes of the mammalian stomach, are aspartic proteases belonging to a broad class of peptidases.

Mucorpepsin (EC 3.4.23.23) is a milk clotting enzyme derived from the fungus *Rhizomucor miehei.*

Commercial relevant milk-clotting enzyme products are often liquid compositions and in the art is described numerous different ways to try to stabilize the milk-clotting enzyme in the product - e.g. to improve storage stability or specific activity of the enzyme.

For instance, EP2333056A1 (DSM, date of fling 12.04.2007) describes that formate, acetate, lactate, propionate, malate, fumarate or propanediol may increase stability of aspartic protease enzyme in a liquid composition/product.

WO2012/127005A1 (DSM) describes a stable liquid chymosin composition comprising inorganic salt in a concentration of 2-100 g/kg and a preservative such as formate, acetate, lactate, propionate, malate, benzoate, sorbate or fumarate, glycol (ethanediol), propylene glycol (propanediol), glycerol, erythritol, xylitol, mannitol, sorbitol, inositol or galactitol. The highest strength of the described chymosin compositions is 1500 IMCU/ml (see e.g. page 6, lines 1-3).

Polyethylene glycol (PEG) is a polymer of ethylene oxide - it may alternatively be termed polyoxyethylene (POE). PEG is commercially available over a wide range of molecular weights such as from 300 g/mol to 10,000,000 g/mol.

DE1492060A1 (Nordmark-Werke GmbH, published in 1969) discloses a method for making a pepsin composition by adding Polyethylene glycol (PEG) with a molecular weight of 400-6000 at a concentration of 1-20 wt% (corresponds to 10000 to 200000 ppm).

**SUMMARY OF THE INVENTION**

A problem to be solved by the present invention is to provide a novel milk clotting aspartic protease enzyme (e.g. chymosin) composition, wherein the aspartic protease has increased physical stability and possibly specific activity.

The solution is based on that the present inventors have identified that by adding suitable polypeptide/protein formulations as set forth in claim 1 to different aspartic protease enzymes, namely chymosin from *Camelius dromedarius,* one significantly improves the physical stability and possibly the specific activity of the enzyme compositions.

Liquid formulations of industrial enzymes are subjected to physical forces (such as shaking) during e.g. transportation and physical stability of an enzyme composition can be tested by repeatable shaking (e.g. via inversion) a sample in a test tube having high head space to sample volume ratio.

As discussed in working Examples herein - to camel chymosin (CHY-MAX® M, Chr. Hansen A/S) were added numerous different polypeptide/protein formulations and a number of these polypeptide/protein formulations significantly increased the physical stability of camel chymosin.

As discussed in working Examples herein - to bovine chymosin (CHY-MAX®, Chr. Hansen A/S), camel chymosin (CHY-MAX® M, Chr. Hansen A/S) and mucorpepsin (Hannilase®, Chr. Hansen A/S) were added numerous different polypeptide/protein formulations and a number of these polypeptide/protein formulations significantly increased the specific activity of the enzyme compositions.

The increase in the specific activity was most significant for the bovine and camel chymosins.

As understood by the skilled person in the present context - specific activity of a milk clotting aspartic protease enzyme composition relates to activity/IMCU per total amount of milk clotting aspartic protease enzyme protein in the composition.

As described in working Examples herein - addition of e.g. whey protein formulations to a purified chymosin sample gave an enzyme composition with significant higher IMCU/ml strength - i.e. a composition with higher specific activity.

For instance, addition of 0,5% (w/w) of whey protein concentrate formulation WPC 80 gave around 10% increase of the strength in the camel chymosin composition as such. Without being limited to theory - it was a surprise to the present inventors that it was possible to increase the strength of e.g. a chymosin composition/product by simply adding a suitable amount of e.g. whey proteins.

It is here relevant to note that casein hydrolysate did not significantly increase the physical stability and/or the specific activity.

As known to the skilled person - in a casein hydrolysate has been performed a hydrolysis of casein.

Without being limited to theory - it is believed that in such a casein hydrolysate formulation a significant amount of the polypeptides are of less than 10 amino acids.

Said in other words and without being limited to theory - it is believed that the herein relevant increased physical stability and specific activity effects are obtained when there are used polypeptides longer than 10 amino acids.

As known in the art - the term peptide may be distinguished from the term protein on the basis of size, which as an arbitrary benchmark may be understood to be approximately 50 or fewer amino acids.

Said in other words, a polypeptide longer than 50 amino acids may normally in the art be understood to be a protein.

Accordingly, a polypeptide longer than 50 amino acids may herein alternatively be termed a protein.

As discussed in working Examples herein - the herein relevant increased/improved physical stability and possibly specific activity effects were related to the amount of polypeptide/protein composition added to chymosin - where no significant positive effect was obtained if less than 0.01% (w/w) was added.

Without being limited to theory - it is believed that addition of polypeptide/protein as set forth in claim 1 provide increased conformational stability to the chymosins and this could explain the observed increased physical stability and possibly increased specific activity observed in working Examples herein.

Without being limited to theory - it is believed that in the prior art it has not been described or suggested that addition of polypeptide/protein may increase the stability of aspartic protease milk-clotting enzymes such as e.g. chymosins - in particular it has not been described that conformational stability may be increased.

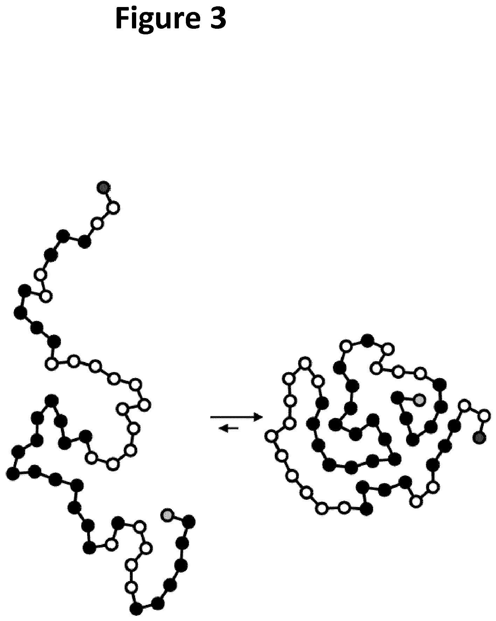

Conformational stability of an enzyme is illustrated in Figure 3 herein.

As known in the art - loss of conformation equals loss of activity of the enzyme - i.e. less specific activity of the enzyme.

Without being limited to theory - loss of conformation may increase denaturation/precipitation of the enzyme and thereby give less physical stability as discussed herein.

As known in the art - milk clotting aspartic protease enzymes may be seen as structurally relatively similar.

As known in the art - different natural wildtype milk clotting aspartic protease polypeptide sequences obtained from different mammalian or fungal species (such as e.g. bovines, camels, sheep, pigs, or mucor) are having a relatively high tertiary structural similarity.

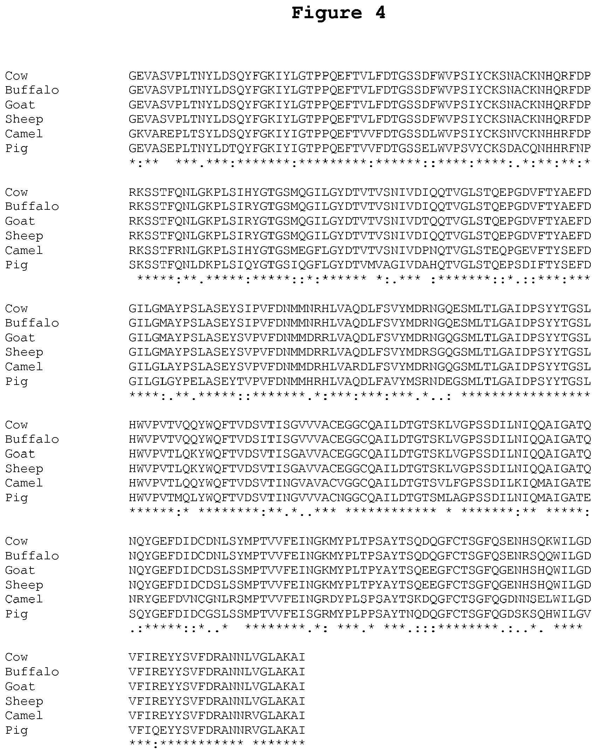

In Figure 4 herein this is provided an alignment of herein relevant different milk clotting chymosin sequences from different mammalian species (cow, buffalo, goat, sheep, camel and pig) - as can be seen in figure 4 they have a close sequence relationship and are known to have a very high tertiary structural similarity.

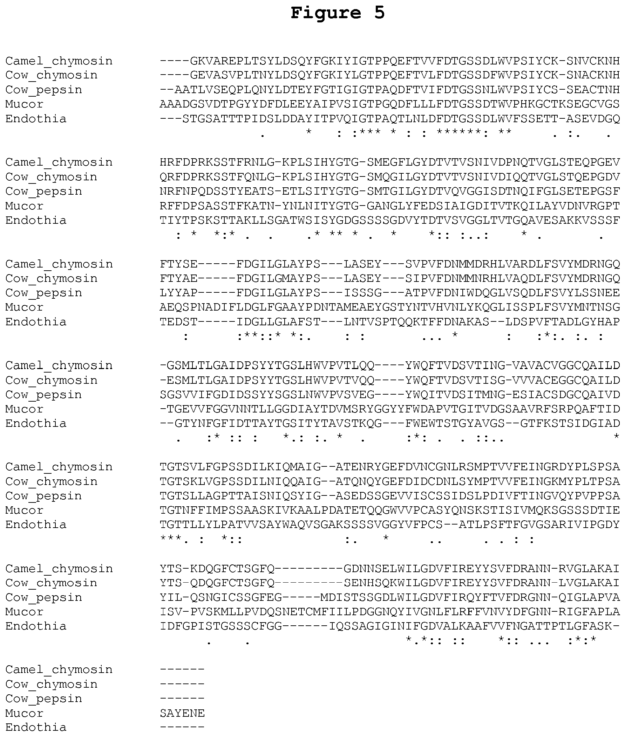

In Figure 5 herein there is provided an alignment of herein relevant commercially available different milk clotting aspartic protease enzymes sequences from different mammalian or fungal species (camel chymosin, cow chymosin, cow pepsin, fungal mucor pepsin and fungal Endothia pepsin).

It may be said that the 5 different sequences of figure 5 are not highly identical - but as known to the skilled person all these 5 different milk clotting aspartic protease enzymes are known to have a high tertiary structural similarity.

As discussed above and shown in working Examples herein - the herein relevant improved/increased stability/activity effects have been demonstrated for bovine chymosin, camel chymosin and mucorpepsin. Only enzyme compositions comprising camel chymosin as set forth in the claims are according to the present invention.

Without being limited to theory - it is believed that there is no significant technical reason to believe that the herein relevant improved/increased stability effect should not be relevant for milk clotting aspartic protease enzymes in general - as discussed above, they are known to have a high tertiary structural similarity and as understood by the skilled person in the present context this tertiary structural similarity makes it plausible that the herein described polymer-enzyme interaction to get improved stability would be a general class effect of the structural similar herein relevant milk clotting aspartic protease enzymes.

As discussed herein, numerous of polypeptide/protein formulations were tested by the present inventors (see working Examples herein) and a number of these significantly increased the specific activity of the enzyme compositions.

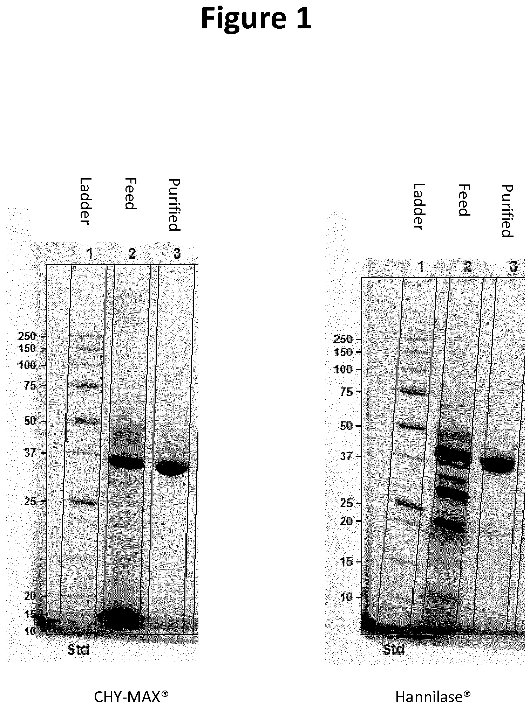

In Figure 1 herein is shown SDS-PAGE data for different samples of bovine chymosin (CHY-MAX®, Chr. Hansen A/S) and mucorpepsin (Hannilase®, Chr. Hansen A/S). For both CHY-MAX® and Hannilase® are Lane 2 (named "Feed") unpurified samples essentially taken from the fermentation media and Lane 3 are purified samples.

In Figure 1 herein can be seen that there are two bands in the migration range 34-38 kDa for e.g. CHY-MAX® in purified sample of lane 3.

Without being limited to theory - it is believed that these two bands represent two different glycosylated forms of CHY-MAX®.

As understood by the skilled person in the present context - both of these different glycosylated forms are milk clotting aspartic protease enzymes, since they both have milk-clotting enzymatic activity.

The term "IMCU/g of the composition" in item I relates to IMCU enzyme activity per gram of the composition as such.

A first aspect of the invention relates to a liquid milk clotting aspartic protease enzyme composition comprising according to claim 1.

In the present context - the skilled person will know or may routinely determine (e.g. based on specific relevant amino acid sequences) the origin of the polypeptides longer than 10 amino acids wherein the polypeptides longer than 10 amino acids are at least one polypeptide selected from the group consisting of: alpha lactalbumin, beta-lactoglobulin, transferrin, lactoperoxidase, casein, alpha-s1-casein, alpha-s2-casein, beta-casein, kappa-casein, ovalbumin, gelatin, soy proteins, pea proteins, corn proteins, potato proteins, hemp proteins, rice proteins, spirulina proteins, wheat proteins, peanut proteins, sun flower proteins, rape seed proteins, blood proteins and algae proteins.

As understood by the skilled person in the present context - the term "g/kg" in relation to item III relates to gram salt per kg of the composition as such.

A second aspect of the invention relates to a liquid milk clotting aspartic protease enzyme composition which must fall within the ambit of claim 1 comprising milk clotting aspartic protease enzyme at a strength of from 1600 IMCU/g to 30000 IMCU/g of the composition and a salt in a concentration from 1 to 350 g/kg and wherein the pH of the composition is from 2 to 8.

A milk clotting aspartic protease enzyme composition as described herein may be used according to the art - e.g. to make a food or feed product of interest (such as e.g. a milk based product of interest that e.g. could be a cheese product).

Accordingly, a third aspect of the invention relates to a method for making a food or feed product according to claim 8 comprising adding an effective amount of a milk clotting aspartic protease enzyme composition of any of first to second aspect or any herein relevant embodiments thereof to the food or feed ingredient(s) and carrying out further manufacturing steps to obtain the food or feed product.

**DEFINITIONS**

All definitions of herein relevant terms are in accordance of what would be understood by the skilled person in relation to the herein relevant technical context.

The term "milk-clotting enzyme" refers to an enzyme with milk-clotting enzymatic activity - i.e. an active milk-clotting enzyme. The milk-clotting activity may be expressed in International Milk-Clotting Units (IMCU) per ml or IMCU per g. The skilled person knows how to determine herein relevant milk-clotting enzymatic activity. In working Example 1 herein is provided an example of a standard method to determine milk-clotting enzymatic activity and specific milk-clotting enzymatic activity. As known in the art - specific clotting activity (IMCU/mg total protein) is determined by dividing the clotting activity (IMCU/ml) by the total protein content (mg total protein per ml).

The term "Sequence Identity" relates to the relatedness between two amino acid sequences.

For purposes of the present invention, the degree of sequence identity between two amino acid sequences is determined according to the art and preferably determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(Identical\ Residues \times 100)/(Length\ of\ Alignment - Total\ Number\ of\ Gaps\ in\ Alignment).$$

The term "variant" means a peptide having milk-clotting enzymatic activity comprising an alteration, *i.e.,* a substitution, insertion, and/or deletion, at one or more (several) positions. A substitution means a replacement of an amino acid occupying a position with a different amino acid; a deletion means removal of an amino acid occupying a position; and an insertion means adding 1-3 amino acids adjacent to an amino acid occupying a position.

The amino acid may be natural or unnatural amino acids - for instance, substitution with e.g. a particularly D-isomers (or D-forms) of e.g. D-alanine could theoretically be possible.

Embodiment of the present invention is described below, by way of examples only.

**DRAWINGS**

Figure 1: In Figure 1 herein is shown SDS-PAGE data for different samples of bovine chymosin (CHY-MAX®, Chr. Hansen A/S) and mucorpepsin (Hannilase®, Chr. Hansen A/S). For both CHY-MAX® and Hannilase® are Lane 2 (named "Feed") unpurified samples essentially taken from the fermentation media and Lane 3 are purified samples.

Figure 2: Shows some of the results of inversion experiments as discussed herein to determine herein relevant physical stability. See working Example herein for further details.

Figure 3: Conformational stability of an enzyme is illustrated.

Figure 4: An alignment of herein relevant different milk clotting chymosin sequences from different mammalian species (cow, buffalo, goat, sheep, camel and pig). All the sequences of figure 4 are public available.

Figure 5: An alignment of herein relevant commercially available different milk clotting aspartic protease enzymes sequences from different mammalian or fungal species (camel chymosin, cow chymosin, cow pepsin, fungal mucor pepsin and fungal Endothia pepsin). All the sequences of figure 5 are public available.

## DETAILED DESCRIPTION OF THE INVENTION

*Milk clotting aspartic protease enzyme*

As discussed in working Examples herein - the herein relevant increase in the specific activity and strength were most significant for the camel chymosin compositions.

The milk clotting aspartic protease enzyme is a *Camelius dromedarius* chymosin (EC 3.4.23.4).

A preferred milk clotting aspartic protease enzyme is *Camelius dromedarius* chymosin as described in e.g. WO02/36752A2 (Chr. Hansen). It may herein alternatively be termed camel chymosin and the publicly known mature polypeptide amino acid sequence is shown in Figure 5 herein.

As known in the art - it is routine work for the skilled person to make variants (i.e. amino acid modifications) of an enzyme of interest without significantly changing the characteristics of the enzyme.

Accordingly, the milk clotting aspartic protease enzyme is *Camelius dromedarius* chymosin comprising the polypeptide amino acid sequence shown in Figure 5 herein (termed "Camel_chymosin") or a variant of *Camelius dromedarius* chymosin, wherein the variant comprises a polypeptide sequence which has at least 90% (preferably at least 95%, more preferably at least 99%) sequence identity with the camel chymosin polypeptide amino acid sequence shown in Figure 5 herein.

*Added polypeptide/proteins*

The polypeptides longer than 10 amino acids are at least one polypeptide selected from the group of polypeptides consisting of: alpha lactalbumin, beta-lactoglobulin, transferrin, lactoperoxidase, casein, alpha-s1-casein, alpha-s2-casein, beta-casein, kappa-casein, ovalbumin, gelatin, soy proteins, pea proteins, corn proteins, potato proteins, hemp proteins, rice proteins, spirulina proteins, wheat proteins, peanut proteins, sun flower proteins, rape seed proteins, blood proteins and algae proteins.

More preferably, the polypeptides longer than 10 amino acids are at least one polypeptide selected from the group of polypeptides consisting of: alpha lactalbumin, beta-lactoglobulin, transferrin, lactoperoxidase, casein, alpha-s1-casein, alphas2-casein, beta-casein, kappa-casein, ovalbumin and gelatin.

Within the group immediately above it is preferred that the polypeptides longer than 10 amino acids are at least one polypeptide selected from the group of polypeptides consisting of: alpha lactalbumin, beta-lactoglobulin, casein, alpha-s1-casein, alpha-s2-casein, beta-casein, kappa-casein, ovalbumin and gelatin.

In a preferred embodiment - polypeptides longer than 10 amino acids are polypeptides longer than 25 amino acids, more preferably polypeptides longer than 40 amino acids.

As discussed in working Examples herein - herein relevant positive results were obtained by addition of e.g. whey protein, ovalbumin and BSA, which herein all may be characterized as relatively large proteins.

As known in the art - the term peptide may be distinguished from the term protein on the basis of size, which as and as an arbitrary benchmark may be understood to be approximately 50 or fewer amino acids.

Said in other words, a polypeptide longer than 50 amino acids may normally in the art be understood to be a protein.

Accordingly, a polypeptide longer than 50 amino acids may herein alternatively be termed a protein.

In a preferred embodiment - polypeptides longer than 10 amino acids are proteins longer than 50 amino acids, more preferably proteins longer than 75 amino acids, even more preferably proteins longer than 150 amino acids.

It may even be preferred that polypeptides longer than 10 amino acids are proteins longer than 300 amino acids.

*First aspect - A liquid milk clotting aspartic protease enzyme composition*

As discussed above - the first aspect of the invention relates to a liquid milk clotting aspartic protease enzyme composition according to claim 1. For the liquid composition - the milk clotting aspartic protease is a camel chymosin.

For the liquid composition - "polypeptides longer than 10 amino acids" are indicated in claim 1.

For the liquid composition - it is preferred that the enzyme strength in item I is a strength of from 100 IMCU/g of the composition to 10000 IMCU/g of the composition, more preferably a strength of from 500 IMCU/g of the composition to 6000 IMCU/g of the composition.

For the liquid composition - in relation to item II, it is preferred the polypeptides longer than 10 amino acids is in a concentration from 0.05% to 8% (w/w) of the composition; more preferably in a concentration from 0.1% to 7% (w/w) of the composition; even more preferably in a concentration from 0.25% to 5% (w/w) of the composition and most preferably in a concentration from 0.5% to 4% (w/w) of the composition (such as e.g. in a concentration from 1% to 3% (w/w) of the composition).

For the liquid composition - the salt in item III is preferably in a concentration from 10 to 300 g/kg, more preferably is in a concentration from 25 to 250 g/kg.

For the liquid composition - it is preferred that the salt is an inorganic salt - preferably wherein the inorganic salt is selected from the group of NaCl, KCl, $Na_2SO_4$, $(NH_4)_2SO_4$. $K_2HPO_4$, $KH_2PO_4$, $Na_2HPO_4$ or $NaH_2PO_4$ or a combination thereof.

Most preferably, the salt is NaCl.

The liquid composition may comprise further additives/compounds such as e.g. a preservative.

As known to the skilled person - preservative may generally be added in a concentration sufficient to prevent microbial growth during shelf life of the product.

Examples of preservatives may be e.g. weak organic acids such as formate, acetate, lactate, propionate, malate, benzoate, sorbate or fumarate. Parabens (alkyl esters of para-hydroxybenzoate) may also be used as preservative. Glycerol or propanediol has also been described as preservatives.

The liquid composition - it is preferred that the pH is from 3 to 7, more preferably that the pH is from 4 to 6.5 and even more preferably that the pH is from 5 to 6.

Preferably, the liquid composition is an aqueous composition, for instance an aqueous solution. As used herein an aqueous composition or aqueous solution encompasses any composition or solution comprising water, for instance at least 20 wt % of water, for instance at least 40 wt % of water.

It may be preferred that the liquid composition as described herein has a total weight of from 10 g to 10000 kg, such as e.g. from 100 g to 3000 kg.

For the liquid composition - a preferred embodiment is:

y: wherein the sum of the amounts of aspartic protease enzyme of item (I) and polypeptides longer than 10 amino acids of item (II), determined by SDS-PAGE, are higher than 50% (w/w) of the total amount of proteins and/or polypeptides longer than 10 amino acids in the composition; more preferably

y: wherein the sum of the amounts of aspartic protease enzyme of item (I) and polypeptides longer than 10 amino acids of item (II), determined by SDS-PAGE, are higher than 70% (w/w) of the total amount of proteins and/or polypeptides longer than 10 amino acids in the composition; even more preferably

y: wherein the sum of the amounts of aspartic protease enzyme of item (I) and polypeptides longer than 10 amino acids of item (II), determined by SDS-PAGE, are higher than 80% (w/w) of the total amount of proteins and/or polypeptides longer than 10 amino acids in the composition; and most preferably

y: wherein the sum of the amounts of aspartic protease enzyme of item (I) and polypeptides longer than 10 amino acids of item (II), determined by SDS-PAGE, are higher than 90% (w/w) of the total amount of proteins and/or polypeptides longer than 10 amino acids in the composition.

In the present context - the skilled person will know or may routinely determine (e.g. based on specific relevant amino acid sequences) the origin of the polypeptides longer than 10 amino acids wherein the polypeptides longer than 10 amino acids of item II are at least one polypeptide selected from the group of polypeptides consisting of: alpha lactalbumin, beta-lactoglobulin, transferrin, lactoperoxidase, casein, alpha-s1-casein, alpha-s2-casein, beta-casein, kappa-casein, ovalbumin, gelatin, soy proteins, pea proteins, corn proteins, potato proteins, hemp proteins, rice proteins, spirulina proteins, wheat proteins, peanut proteins, sun flower proteins, rape seed proteins, blood proteins and algae proteins.

Accordingly, the skilled person may therefore also routine determine (e.g. via SDS-PAGE) if item y is fulfilled in a herein relevant composition of interest.

*Second aspect*

As discussed above - the second aspect of the invention relates to a liquid milk clotting aspartic protease enzyme composition which must fall within the ambit of claim 1 comprising milk clotting aspartic protease enzyme at a strength of from 1600 IMCU/g to 30000 IMCU/g of the composition and a salt in a concentration from 1 to 350 g/kg and wherein the pH of the composition is from 2 to 8.
For the liquid composition - preferably, the strength is a strength of from 2000 IMCU/g to 15000 IMCU/g of the composition, such as from 3000 IMCU/g to 10000 IMCU/g of the composition or such as from 4000 IMCU/g to 8000 IMCU/g of the composition.

*Third aspect - A method for a method for making a food or feed product*

As discussed above - a milk clotting aspartic protease enzyme composition as described herein may be used according to the art - e.g. to make a milk based product of interest (such as e.g. a cheese product).
As discussed above - the third aspect of the invention relates to a method for making a food or feed product according to claim 8 comprising adding an effective amount of a milk clotting aspartic protease enzyme composition of the first or second aspect to the food or feed ingredient(s) and carrying out further manufacturing steps to obtain the food or feed product.
Preferably, the food or feed product is a milk based product and wherein the method comprises adding an effective amount of the composition according to claim 1 to milk and carrying our further manufacturing steps to obtain the milk based product.
The milk may e.g. be sheep milk, goat milk, buffalo milk, yak milk, lama milk, camel milk or cow milk.
The milk based product may e.g. be a fermented milk product, a quark or a cheese.

**EXAMPLES**

**EXAMPLE 1: Determination of specific milk-clotting activity**

4.1 Determination of clotting activity

Milk clotting activity was determined using the REMCAT method, which is the standard method developed by the International Dairy Federation (IDF method).
Milk clotting activity is determined from the time needed for a visible flocculation of a standard milk substrate prepared from a low-heat, low fat milk powder with a calcium chloride solution of 0,5 g per litre (pH ≈ 6,5). The clotting time of a milk-clotting enzyme sample is compared to that of a reference standard having known milk-clotting activity and having the same enzyme composition by IDF Standard 110B as the sample. Samples and reference standards were measured under identical chemical and physical conditions. Variant samples were adjusted to approximately 3 IMCU/ml using an 84 mM acetic acid pH 5.5 buffer. Hereafter, 200 µl enzyme was added to 10 ml preheated milk (32°C) in a glass test tube placed in a water bath, capable of maintaining a constant temperature of 32°C ± 1°C under constant stirring.
The total milk-clotting activity (strength) of a milk-clotting enzyme is calculated in International Milk-Clotting Units (IMCU) per ml relative to a standard having the same enzyme composition as the sample according to the formula:

$$\text{Strength in IMCU/ml} = \frac{S_{standard} \times T_{standard} \times D_{sample}}{D_{standard} \times T_{sample}}$$

Sstandard: The milk-clotting activity of the international reference standard for rennet.
Tstandard: Clotting time in seconds obtained for the standard dilution.
Dsample: Dilution factor for the sample
Dstandard: Dilution factor for the standard
Tsample: Clotting time in seconds obtained for the diluted rennet sample from addition of enzyme to time of flocculation

4.2 Determination of total protein content

Total protein content was determined using the Pierce BCA Protein Assay Kit from Thermo Scientific following the instructions of the providers.

4.3 Calculation of specific clotting activity

Specific clotting activity (IMCU/mg total protein) was determined by dividing the clotting activity (IMCU/ml) by the total protein content (mg total protein per ml).

## EXAMPLE 2: Enzyme preparations

Bovine chymosin (CHY-MAX®, Chr. Hansen A/S) or camel chymosin (CHY-MAX® M, Chr. Hansen A/S) were recombinantly expressed in *Aspergillus niger* (roughly as described in WO02/36752A2). The enzymes were purified by chromatography technology. Mucorpepsin (Hannilase®, Chr. Hansen A/S) derived from *Rhizomucor miehei* was produced by use of *Rhizomucor miehei* as production host cell and purified by chromatography technology. For all the purified enzyme samples - at least 90% of the total amounts of proteins with a size bigger than 10 kDa, determined by SDS-PAGE, in the purified sample were the relevant milk clotting aspartic protease enzymes.

All enzyme samples were prepared by mixing an exact volume for a stock solution of the enzyme with a solution of the additive and adding buffer to a final volume. In this manner the concentration of enzyme protein is kept constant for all prepared samples. Buffer composition was: 0.25 M sodium acetate, 20 mM sodium phosphate, 2.0 M sodium chloride, pH 5.7, 5 mM methionine, and 35 mM sodium benzoate. The strength was from 200 to 1200 IMCU/ml. The composition was added different polypeptides such as Hammersten casein, WPC80, Lacprodan Alpha 10, Lacprodan Alpha 20, etc.

WPC80 is a commercial preparation of dried whey protein concentrate with 80% protein. Lacprodan Alpha 10 and Lacprodan Alpha 20 are commercial preparations of whey protein isolate containing 43% and 60% alpha lactalbumin of total protein content, respectively.

## EXAMPLE 3: Physical stability of camel chymosin

Liquid formulations of industrial enzymes are subjected to physical forces from unit operations such as pumping, stirring and filtration over membranes. During transportation of partly filled containers sloshing around of liquid formulation may also contribute to this. Shear stress and increased exposure of enzyme to the water-air interface may induce denaturation and concomitant loss of enzyme activity.

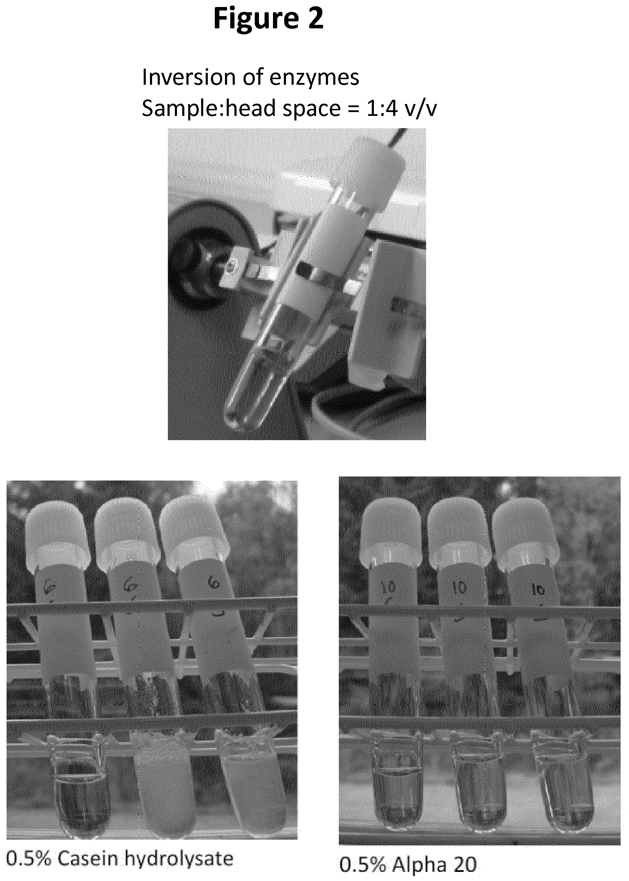

Physical stability of an enzyme or protein sample can be tested by repeatable shaking a sample of the enzyme in a test tube having high head space to sample volume ratio. The stability of different aspartic proteases towards shaking was investigated by inverting a 2 ml sample filled in a 10 ml tube in a rotary device for 1 hour (see Figure 2 herein). For each solution, relative milk clotting activity was measured after 1 hour of vertical inversion (at room temperature) and compared to a non-inverted control having the exact same composition. Results were expressed as "retained activity" which is obtained by diving activity of the inverted sample with the activity of the non-inverted control sample.

Results:

Vertical inversion for 1 hr of a sample of camel chymosin results in an activity loss of more than 30% cf. Table 1 (No addition). Addition of PEG8000 to a concentration of 0.015% was found to protect camel chymosin and resulting in no loss of activity upon vertical inversion. When the sample of camel chymosin contained Hammersten casein, Alpha 10, Alpha 20 or WPC 80 at a concentration of either 0.5% w/v or 1.0% w/v practically no loss upon vertical inversion was seen.

The protecting effect of Alpha 20 and WPC 80 was found to decrease gradually when their concentration was decreased below 0.1%. A formulation of camel chymosin with either acid casein hydrolysate or whey permeate did not increase stability of the enzyme as the loss in activity upon vertical inversion was the same as for the untreated control sample (Table 3).

Conclusion: The results show that certain proteins added to a preparation of camel chymosin can increase the physical stability of the enzyme.

Table 1: Retained activity of camel chymosin samples subjected to vertical inversion for 1 hr (Internal ref number: EXP-13-AD2681).

| Compound | Retained activity |
|---|---|
| No addition | 67% (3%) |
| PEG (0.015%) | 99% (0%) |
| Glycerol (6%) | 67% (4%) |

(continued)

| Compound | Retained activity |
|---|---|
| 0.5% Hammersten casein | 97% (0%) |
| 1% Hammersten casein | 97% (1%) |
| 0.5% Casein hydrolysate (Merck) | 67% (4%) |
| 1% Casein hydrolysate (Merck) | 74% (5%) |
| 0.5% Alpha 10 (Prøve 1) | 98% (0%) |
| 1% Alpha 10 (Prove 1) | 98% (0%) |
| 0.5% Alpha 20 (Prove 2) | 98% (0%) |
| 1% Alpha 20 | 98% (0%) |
| 0.5% Permeat | 64% (17%) |
| 1% Permeat | 69% (1%) |
| 0.1% WPC 80 | 100% (0%) |
| 1% WPC 80 | 99% (0%) |

**EXAMPLE 4: Specific activity of camel chymosin**

Results:

The inversion experiments were designed so the exact same amount of enzyme protein was added in each experiment and all formulations were made up to the same volume. If composition of the formulation did not influence enzymatic activity, one would expect to find the same enzymatic activity of all control samples, i.e. samples not inverted. However, this was not the case. Samples containing PEG8000 were 4-5% higher in activity in good accordance with recently submitted patent application IN5103DK00. Samples containing Hammerstein casein, Alpha 20 or WPC 80 had 13 - 18% higher activity compared to the sample without additives (no addition) even though the same amount of enzyme protein was applied (Table 2). This shows that the presence milk proteins in the formulation of camel chymosin increase the specific activity of the enzyme. The same conclusion is made from Table 3 which shows the activity of compostions containing Alpha 20 and WPC80 at five different concentrations. Table 5 show activity of the composition, activity index with 'no addition' = 100%, and the retained activity of a sample subjected to vertical inversion for 1 hr.

Addition of polypeptides to a composition of camel chymosin has two effects on the enzyme: Increase in specific activity and an increased physical stability of the enzyme. Both properties correlate well with the dosage of polypeptide as seen from Table 3. In Table 3 it is found that when the concentration of Alpha 20 is decreased from 0.5 to 0.01%, the enzyme activity drops from index 115 to index 102 which is almost the same level as the untreated control. At a concentration of 0.01% Alpha 20 the physical stability (retained activity) of the enzyme is the same as in the control experiment (no addition).

Table 2: Activity of CHY-MAX® M added different polypeptides. Activity index relative to untreated control ('no addition') is shown in percentage (Internal ref number: EXP-13-AD2681).

| Compound | Activity (IMCU/ml) | |
|---|---|---|
| No addition | 1124 (13.3) | 100% |
| PEG (0.015%) | 1163 (6.8) | 104% |
| Glycerol (6%) | 1069 (12.0) | 95% |
| 0.5% Hammersten casein | 1251 (12.3) | 111% |
| 1% Hammersten casein | 1275 (25.0) | 113% |
| 0.5% Casein hydrolysate (Merck) | 1112 (12.3) | 99% |
| 1% Casein hydrolysate (Merck) | 1102 (19.8) | 98% |
| 0.5% Alpha 10 (Prøve 1) | 1282 (8.6) | **114%** |

(continued)

| Compound | Activity (IMCU/ml) | |
|---|---|---|
| 1% Alpha 10 (Prøve 1) | 1305 (9.9) | **116%** |
| 0.5% Alpha 20 (Prøve 2) | 1287 (5.0) | **115%** |
| 1% Alpha 20 (Prøve 2) | 1323 (1.2) | **118%** |
| 0.5% Permeat (Prøve 3) | 1147 (15.6) | 102% |
| 1% Permeat (Prøve 3) | 1127 (32.2) | 100% |
| 0.1% WPC 80 (Prøve 4) | 1248 (15.3) | **111%** |
| 1% WPC 80 (Prøve 4) | 1287 (2.8) | **115%** |

Table 3: Activity of CHY-MAX® M added different polypeptides. Activity index relative to untreated control ('no addition') is shown in percentage. Column to the right show retained activity of camel chymosin samples subjected to vertical inversion for 1 hr (Internal ref number: EXP-13-AD2681).

| Compound | Activity (IMCU/ml) | | Retained activity |
|---|---|---|---|
| No addition | 1102 (19.6) | 100% | 76% (4%) |
| PEG, 0.015% | 1153 (12.4) | 105% | 101% (2%) |
| Alpha 20, 0.5% | 1269 (13.0) | 115% | 100% (1%) |
| Alpha 20, 0.25% | 1224 (8.5) | 111% | 100% (1%) |
| Alpha 20, 0.1 | 1162 (1.2) | 105% | 100% (0%) |
| Alpha 20, 0.05% | 1134 (7.2) | 103% | 97% (0%) |
| Alpha 20, 0.01% | 1129 (1.0) | 102% | 79% (0%) |
| WPC 80, 0.5% | 1235 (13.9) | 112% | 101% (1%) |
| WPC 80, 0.25% | 1227 (10.2) | 111% | 98% (0%) |
| WPC 80, 0.1% | 1155 (14.3) | 105% | 100% (1%) |
| WPC 80, 0.05% | 1141 (7.3) | 104% | 95% (2%) |
| WPC 80, 0.01% | 1112 (5.4) | 101% | 80% (1%) |
| **Conclusion:** Addition of polypeptides to a composition of camel chymosin has two effects on the enzyme: Increase in specific activity and an increased physical stability of the enzyme. Both properties correlate well with the dosage of polypeptide. | | | |

## EXAMPLE 5: Specific activity of camel chymosin, bovine chymosin, mucor pepsin L and mucor pepsin XL

Table 4: Activity of milk clotting enzymes added different polypeptides (Internal ref number: EXP-13-AD2690). Standard deviation in absolute numbers is in parenthesis.

| Compound | CHY-MAX® M | | CHY-MAX ® | | Hannilase L | | Hannilase XP | |
|---|---|---|---|---|---|---|---|---|
| Control | 1066 (18.7) | 100% | 1117 (11.7) | 100% | 985 (4.0) | 100% | 1041 (2.3) | 100% |
| *Alpha 20, 0.1%* | *1108 (6.2)* | 104% | *1143 (2.7)* | 102% | 983 (9.4) | 99% | *1035 (7.1)* | 100% |
| Alpha 20, 0.5% | 1192 (201) | **111%** | 1173 (3.3) | **105%** | 994 (7.6) | 100% | 1044 (2.3) | 100% |

(continued)

| Compound | CHY-MAX® M | | CHY-MAX ® | | Hannilase L | | Hannilase XP | |
|---|---|---|---|---|---|---|---|---|
| Alpha 20, 10% | 1225 (6.8) | **116%** | 1191 (3.0) | **107%** | 1,007 (11.0) | 101% | 1057 (5.1) | 102% |
| WPC80 0.1% | 1086 (11.4) | 103% | 1131(9.1) | 101% | 980 (10.2) | 101% | 1030 (13.5) | 100% |
| WPC80 0.5% | 1156 (11.0) | **110%** | 1151 (7.2) | 102% | 995 (9.8) | 101% | 1034 (25) | 99% |
| WPC80 10% | 1196 (205) | **114%** | 1170 (15.6) | **104%** | 997 (3.7) | 102% | 1047 (9.7) | 101% |
| Conclusion: The results in table 2-4 show that addition of polypeptides to a formulation of aspartic proteases increase the specific activity. | | | | | | | | |

**Example 6:**

The table below show results from tests performed similar to Example 3 above - but using different protein formulations. All proteins shown in the table were added to a final content of 1% w/w and with gliadin as only exception gave clear solutions. Physical stability was tested according to example 3. Samples were stored for 1 year at 5°C and 37°C, respectively, and stability was tested during storage period. The column 'End of storage' shows remaining activity after 1 year - the number was calculated by fitting a single exponential function to all data points.

As known in the art - the term "peptone" refers to proteins digested by proteolysis. As known in the art - the term "tryptone" refers to proteins digested by the protease trypsin.

Table 5: Activity of CHY-MAX M added different polypeptides at 1% w/w. Activity index relative to untreated control ('no addition') is shown in percentage. Column 'Retained Activity' show retained activity of camel chymosin samples subjected to vertical inversion for 1 hr. Column 'End of Storage' show activity after storage at 5°C and 37°C. Standard deviation is shown in parenthesis. (Internal ref number: EXP-14-AE8307)

| | Compound | Activity IMCU/ml / Index | | Retained activity | End of storage (5°C / 37 °C) |
|---|---|---|---|---|---|
| 1 | Control (no addition) | 389 (3.8) | 100% | 36% (2.4) | 101% / 14% |
| 2 | 18332 Peptone (vegetable) | 400 (11.1) | 106% | 95% (3.8) | 91% / 13% |
| 3 | 51841 Peptone (vegetable) acid hydrolysate | 418 (1.5) | 109% | 99% (4.6) | 94% / 13% |
| 4 | 19942 Peptone (vegetable), no 1 | 422 (5.5) | 108% | 97% (0.6) | 93% / 15% |
| 5 | 61854 Peptone (vegetable), no 2 | 423 (3.7) | 109% | 99% (1.4) | 93% / 13% |
| 6 | 92976 Peptone special (vegetable) | 423(1.8) | 110% | 93% (7.1) | 93% / 13% |
| 7 | 29185 Proteose Peptone (vegetable) | 428 (3.0) | 110% | 97% (0.6) | 94% / 12% |
| 8 | 16922 Tryptone (vegetable) | 431 (2.9) | 111% | 96% (0.6) | 93% / 13% |
| 9 | 12331 Tryptose (vegetable) | 428 (2.0) | 111% | 94% (5) | 96% / 15% |
| 10 | 05138 Vegetable Extract | 412 (9.8) | 109% | 98% (2.3) | 100% / 14% |
| 11 | 04316 Vegetable Extract no 1 | 414 (3.1) | 108% | 97% (5.1) | 97% / 15% |
| 12 | 49869 Vegetable Extract no 2 | 409 (23) | 106% | 99% (3.5) | 97% / 15% |
| 13 | 07436 Vegetable hydrolysate no 2 | 411 (5.8) | 107% | 101% (25) | 98% / 14% |

(continued)

| | Compound | Activity IMCU/ml / Index | | Retained activity | End of storage (5°C / 37 °C) |
|---|---|---|---|---|---|
| 14 | 67381 Vegetable Infusion powder | 403 (5.9) | 103% | 99% (2.9) | 102% / 13% |
| 15 | 95757 Vegetable Special Infusion powder | 411 (16) | 106% | 98% (6.4) | 99% / 18% |
| 16 | 83059 Peptone from potatoes | 403 (1.9) | 104% | 99% (1.5) | 100% / 13% |
| 17 | 93491 Peptone from broad bean | 425 (10.2) | 108% | 99% (1.4) | 96% / 12% |
| 18 | 93492 Peptone from wheat | 427 (1.2) | 110% | 100% (2.2) | 100% / 13% |
| 19 | 90765 Peptone from soybean, enzymatic digest | 408 (11.2) | 108% | 100% (2.3) | 102% / 18% |
| 20 | 70178 Peptone from soybean, enzymatic digest | 401 (0.3) | 104% | 92% (23) | 98% / 14% |
| 21 | S1674 Soy protein acid hydrolysate | 432 (13.7) | 109% | 88% (1.3) | 106% / 18% |
| 22 | 87972 Peptone from soybean, enzymatic digest | 400 (7.0) | 102% | 91% (0.6) | 106%/21% |
| 23 | 96174 Peptone from pea | 404 (2.9) | 104% | 98% (1.3) | 98% / 10% |
| 25 | 49760 GLUTEN HYDROLYSATE F. MAIZE | 432 (5.1) | 111% | 95% (2) | 107% / 16% |
| 26 | Ovalbumin | 464 (4.9) | 121% | 99% (1.1) | 97% / 14% |
| 27 | Gelatin | 475 (6.0) | 124% | 99% (1.7) | 101% / 16% |
| 28 | Bovine serum albumin | 487 (81) | 128% | 100% (2.6) | 104% / 15% |
| 29 | PEG8000 (0.015%) | 443 (1.8) | 115% | 98% (0.1) | 104% / 15% |

**Example 7:**

Extracts of plant proteins were prepared by suspending 2 g sample in 40 ml brine consisting of: 12 % NaCl, 20 g/L NaAc anhydrous, 2.5 g/L NaH2PO4 anhydrous, and 10 g/L Na-benzoate in water, pH 5.4 - 5.8. After mixing for 2 hours on rotating device the suspension was centrifuged and the supernatant pH adjusted to 5.4 - 5.8 and filtered through a 0.45 μm syringe filter. The extracts were used for preparing formulations of CHY-MAX M by mixing with an exact measured and equal volume of a stock solution the enzyme to give samples having same concentration of enzyme proteins. In this example, extracts of 27 different plant proteins were tested in three groups with each group prepared on different days.

The activity was measured one day after sample preparation; the column titled activity shown activity in IMCU/ml and relative to untreated control (no addition). Physical stability was tested according to example 3 with the only difference that in present example a different rotary device was used for vertical inversion of the samples (Multi RS-60 from Biosan at 32 rpm for 1 hour). The change in rotary device may have resulted in increased physical stress of the samples since retained activity of untreated sample was only 10% compared to ca. 70% in preceding examples. Samples were stored for 1 year at 5°C and 37°C, respectively, and stability was tested during storage period. The column 'End of storage' shows remaining activity after 1 year - the number was calculated by fitting a single exponential function to all data points.

**Table 6:** Activity of CHY-MAX® M added different polypeptides. Activity index relative to untreated control ('no addition') is shown in percentage. Column 'Retained Activity' show retained activity of camel chymosin samples subjected to vertical inversion for 1 hr. Column 'End of Storage' show activity after storage at 5°C and 37°C. Standard deviation is shown in parenthesis. Internal ref number: Group 1 / EXP-14-AF3604.

| | Compound | Activity IMCU/ml / Index | | Retained activity | End of storage (5°C / 37 °C) |
|---|---|---|---|---|---|
| | No addition (only Brine) | 405 (6.8) | 100% | 10% (1.4) | 96% / 22% |
| | 0.015% PEG 8000 | 481.9 (6.5) | 119% | 99% (3.1) | 95%/21% |
| 1 | Nutralys F 85 F (Roquette) | 450.4 (6) | 111% | 96% (1) | 87% / 17% |
| 2 | Nutralys F85G (Roquette) | 421.6 (4.6) | 104% | 102% (0.5) | 91%/20% |
| 3 | Nutralys F 85 M (Roquette) | 419.9 (13.8) | 104% | 102% (0.9) | 89%/10% |
| 4 | Nutralys S 85 F (Roquette) | 415.2 (6.2) | 103% | 102% (0.2) | 87% / 19% |
| 5 | Nutralys W (Roquette) | 461.3 (11.3) | 114% | 100% (0.4) | 87% / 14% |
| 6 | Solulys 048E (Roquette) | 399.5 (12.4) | 99% | 103% (1) | 94% / 8% |
| 7 | Solulys 095E (Roquette) | 412.3 (2.6) | 102% | 100% (1.8) | 91%/9% |
| 8 | Tubermine Fv Proteine De Pomme Des Terre (Roquette) | 403.4 (5.7) | 100% | 96% (0) | 94% / 15% |
| 9 | Tubermine GP Proteine De Pomme Des Terre (Roquette) | 411 (3.3) | 101% | 90% (0.7) | 95% / 17% |

**Table 7:** Activity of CHY-MAX®M added different polypeptides. Activity index relative to untreated control ('no addition') is shown in percentage. Column 'Retained Activity' show retained activity of camel chymosin samples subjected to vertical inversion for 1 hr. Column 'End of Storage' show activity after storage at 5°C and 37°C. Standard deviation is shown in parenthesis. Internal ref number: Group 2 / EXP-14-AF3604.

| | Compound | Activity IMCU/ml / Index | | Retained activity | End of storage (5°C / 37 °C) |
|---|---|---|---|---|---|
| | No addition (only Brine) | 395.9 (10.8) | 100% | 7% (1.25) | 93% / 24% |
| | No addition (only Brine) | 385 (3.7) | 97% | 7.5% (0.49) | - |
| | No addition (only Brine) | 396.3 (4.9) | 100% | 6.3% (0.54) | - |
| | 0.015% PEG 8000 | 457.8 (5.6) | 116% | 98.9% (1.13) | 99%/24% |
| 10 | Alburex E 361 G (Roquette) | 511.1 (1) | **129%** | 92.7% (0.84) | 95%/21% |
| 11 | Gluten De Ble Supra Vital (Roquette) | 423.2 (2.2) | 107% | 90% (2.83) | 92% / 17% |
| 12 | Lysamine GP Proteine De Pois (Roquette) | 391.4 (13.6) | 99% | 77.3% (0.88) | 95% / 14% |
| 13 | Corn Gluten (Roquette) | 359.1 (18.7) | 91% | 97.3% (0.28) | 103% / 22% |
| 14 | Ærteprotein (Naturdrogeriet A/S) | 440.4 (3.8) | 111% | 98.3% (1.36) | 86% / 19% |
| 15 | Sojaprotein (Naturdrogeriet A/S) | 427 (1.1) | 108% | 106.8% (6.59) | 88% / 17% |
| 16 | Vegan Ris protein (Naturdrogeriet A/S) | 352.4 (4.3) | 89% | 96.5% (2.22) | 114%/28% |
| 17 | Sojaprotein Keto lyse A/S | 437 (6) | 110% | 94.2% (2.41) | 89% / 18% |
| 18 | Hamp protein complex økologisk (Nybogaard A/S) | 422.7 (3.2) | 107% | 98.2% (2.66) | 100% /20% |

**Table 8:** Activity of CHY-MAX® M added different polypeptides. Activity index relative to untreated control ('no addition') is shown in percentage. Column 'Retained Activity' show retained activity of camel chymosin samples subjected to vertical inversion for 1 hr. Column 'End of Storage' show activity after storage at 5°C and 37°C. Standard deviation is shown in parenthesis. Internal ref number: Group 3 / EXP-14-AF3604.

| | Compound | Activity IMCU/ml / Index | | Retained activity | End of storage (5°C / 37 °C) |
|---|---|---|---|---|---|
| | No addition (only Brine) | 419.5 (1.1) | 100% | 8.8% (2.38) | 97% / 28% |
| | 0.015% PEG 8000 | 478.1 (2.9) | 114% | 102.8% (1.12) | 100% / 29% |
| 19 | Spirulina pulver økologisk (Dinsundhed.Net Aps) | 484.8 (9.4) | **116%** | 97% (0.17) | 57% / 1% |
| 20 | Plant force rice protein (Third Wave Nutrition) | 413.4 (5) | 99% | 87.5% (1.45) | 96% / 17% |
| 21 | Superfruit Hemp protein (Superfruit Scandinavia AB) | 447.8 (4.1) | 107% | 97.7% (1.81) | 97% / 20% |
| 22 | Hampe protein økologisk (Nybogaard A/S) | 421.2 (1.3) | 100% | 101.2% (2.12) | 98% / 23% |
| 23 | Plant force Rice protein (Third Wave Nutrition) | 412.7 (9.8) | 98% | 84.1% (5.77) | 99% / 18% |
| 24 | Nutralys T 65 M (Roquette) | 454.5 (8.5) | 108% | 100.1% (0.34) | 88% / 14% |
| 25 | LAB 4460 Nutralys PEA XF EXP (Roquette) | 451.9 (13.1) | 108% | 97.2% (0.1) | 90% / 19% |
| 26 | LAB 4462 Nutralys PEA BF EXP (Roquette) | 436.8 (10.5) | 104% | 99.9% (0.45) | 93% / 16% |
| 27 | Gluten De MAIS 58E (Roquette) | 402.9 (0.5) | 96% | 65.5% (1.33) | 97% / 20% |

**REFERENCES**

1: EP2333056A1 (DSM, date of fling 12.04.2007)
2: WO2012/127005A1 (DSM)
3: DE1492060A1 (Nordmark-Werke GmbH, published in 1969)

**Claims**

1. A liquid milk clotting aspartic protease enzyme composition comprising:

I: milk clotting aspartic protease enzyme at a strength of from 25 IMCU/g to 30000 IMCU/g of the composition;
II: polypeptides longer than 10 amino acids in a concentration from 0.01% to 10% (w/w) of the composition, wherein the polypeptides longer than 10 amino acids are at least one polypeptide selected from the group consisting of: alpha lactalbumin, beta-lactoglobulin, transferrin, lactoperoxidase, casein, alpha-s1-casein, alpha-s2-casein, beta-casein, kappa-casein, ovalbumin, gelatin, soy proteins, pea proteins, corn proteins, potato proteins, hemp proteins, rice proteins, spirulina proteins, wheat proteins, peanut proteins, sun flower proteins, rape seed proteins, blood proteins and algae proteins; and
III: a salt in a concentration from 1 to 350 g/kg and wherein the pH of the composition is from 2 to 8; and wherein the milk clotting aspartic protease enzyme is chymosin (EC 3.4.23.4),
wherein the milk clotting aspartic protease enzyme is *Camelius dromedarius* chymosin comprising the polypep-

EP 3 167 056 B1

tide amino acid sequence shown in Figure 5 herein (termed "Camel_chymosin") or a variant of *Camelius dromedarius* chymosin, wherein the variant comprises a polypeptide sequence which has at least 90%, preferably at least 95%, more preferably at least 99%, sequence identity with the camel chymosin polypeptide amino acid sequence shown in Figure 5 herein, wherein the International Milk Clotting Units (IMCU) are determined as set forth in the description.

2. The liquid milk clotting aspartic protease enzyme composition of claim 1, wherein in relation to item II), the polypeptides longer than 10 amino acids is in a concentration from 0.05% to 8% (w/w) of the composition; preferably in a concentration from 0.1% to 7% (w/w) of the composition; more preferably in a concentration from 0.25% to 5% (w/w) of the composition and even more preferably in a concentration from 0.5% to 4% (w/w) of the composition, such as e.g. in a concentration from 1% to 3% (w/w) of the composition.

3. The liquid milk clotting aspartic protease enzyme composition of claim 1, wherein the salt in item (iii) is in a concentration from 10 to 300 g/kg, preferably is in a concentration from 25 to 250 g/kg.

4. The liquid milk clotting aspartic protease enzyme composition of claim 1, wherein the salt is an inorganic salt - preferably wherein the inorganic salt is selected from the group of NaCl, KCl, $Na_2SO_4$, $(NH_4)_2SO_4$, $K_2HPO_4$, $KH_2PO_4$, $Na_2HPO_4$ or $NaH_2PO_4$ or a combination thereof, preferably the salt is NaCl.

5. The liquid milk clotting aspartic protease enzyme composition of claim 1 comprising a preservative, preferably weak organic acids such as formate, acetate, lactate, propionate, malate, benzoate, sorbate or fumarate; or parabens such as alkyl esters of parahydroxybenzoate; or glycerol or propanediol.

6. The liquid milk clotting aspartic protease enzyme composition of claim 1, wherein the pH of the composition is from 3 to 7, preferably the pH is from 4 to 6.5, more preferably the pH is from 5 to 6.

7. A liquid milk clotting aspartic protease enzyme composition of claim 1,

wherein the concentration of added polypeptides in item II is in a concentration from 0.5% to 4% (w/w) of the composition; and
wherein the liquid composition has a total weight of from 100 g to 3000 kg; and
(y): wherein the sum of the amounts of aspartic protease enzyme of item I and polypeptides longer than 10 amino acids of item II, determined by SDS-PAGE, are higher than 50% (w/w) of the total amount of proteins and/or polypeptides longer than 10 amino acids in the composition; and
wherein the polypeptides longer than 10 amino acids are at least one polypeptide selected from the group of polypeptides consisting of: alpha lactalbumin, beta-lactoglobulin, transferrin, lactoperoxidase, casein, alpha-s1-casein, alpha-s2-casein, beta-casein, kappa-casein, ovalbumin and gelatin.

8. A method for making a food or feed product comprising adding an effective amount of a milk clotting aspartic protease enzyme composition of any of the previous claims to the food or feed ingredient(s) and carrying out further manufacturing steps to obtain the food or feed product.

**Patentansprüche**

1. Flüssige Milchgerinnungs-Aspartatprotease-Enzymzusammensetzung, umfassend:

I: Aspartatprotease-Enzym in einer Stärke von 25 IMCU/g bis 30000 IMCU/g der Zusammensetzung;
II: Polypeptide länger als 10 Aminosäuren in einer Konzentration von 0,01 % bis 10 % (Gew.-%) der Zusammensetzung, wobei die Polypeptide länger als 10 Aminosäuren mindestens ein Polypeptid ausgewählt aus der Gruppe bestehend aus Folgendem sind: alpha-Lactalbumin, beta-Lactoglobulin, Transferrin, Lactoperoxidase, Casein, alpha-s1-Casein, alpha-s2-Casein, beta-Casein, kappa-Casein, Ovalbumin, Gelatine, Sojaproteine, Erbsenproteine, Maisproteine, Kartoffelproteine, Hanfproteine, Reisproteine, Spirulinaproteine, Weizenproteine, Erdnussproteine, Sonnenblumenproteine, Rapsproteine, Blutproteine und Algenproteine; und
III: ein Salz in einer Konzentration von 1 bis 350 g/kg und
wobei der pH-Wert der Zusammensetzung 2 bis 8 beträgt; und
wobei das Milchgerinnungs-Asparagin-Protease-Enzym Chymosin (EC 3.4.23.4) ist, wobei das Milchgerinnungs-Asparagin-Protease-Enzym *Camelius dromedarius*-Chymosin, das die in Figur 5 gezeigte Polypeptid-Aminosäuresequenz (genannt "Camel_chymosin") umfasst, oder eine Variante von *Camelius dromedarius-Chymosin* ist, wobei die Variante eine Polypeptidsequenz umfasst, die mindestens 90 %, bevorzugt mindestens 95 %, mehr bevorzugt mindestens 99 % Sequenzidentität mit der hier in Figur 5 gezeigten Kamel-Chymosin-Polypeptid-Aminosäuresequenz aufweist, wobei die International Milk Clotting Units (IMCU) wie in der Beschreibung angegeben bestimmt werden.

2. Flüssige Milchgerinnungs-Aspartatprotease-Enzymzusammensetzung nach Anspruch 1, wobei in Bezug auf Punkt II) die Polypeptide länger als 10 Aminosäuren in einer Konzentration von 0,05 % bis 8 % (Gew.-%) der Zusammensetzung; bevorzugt in einer Konzentration von 0,1 % bis 7 % (Gew.-%) der Zusammensetzung, mehr bevorzugt in einer Konzentration von 0,25 % bis 5 % (Gew.-%) der Zusammensetzung und noch mehr bevorzugt in einer Konzentration von 0,5 % bis 4 % (Gew.-%) der Zusammensetzung, wie z.B. in einer Konzentration von 1 % bis 3 % (Gew.-%) der Zusammensetzung, vorliegen.

3. Flüssige Milchgerinnungs-Aspartatprotease-Enzymzusammensetzung nach Anspruch 1, wobei das Salz in Punkt (iii) in einer Konzentration von 10 bis 300 g/kg, bevorzugt in einer Konzentration von 25 bis 250 g/kg, vorliegt.

4. Flüssige Aspartatprotease-Enzymzusammensetzung nach Anspruch 1, wobei das Salz ein anorganisches Salz ist - wobei das anorganische Salz bevorzugt aus der Gruppe von $NaCl$, $KCl$, $Na_2SO_4$, $(NH_4)_2SO_4$, $K_2HPO_4$, $KH_2PO_4$, $Na_2HPO_4$ or $NaH_2PO_4$ oder einer Kombination davon ausgewählt ist, das Salz bevorzugt $NaCl$ ist.

5. Flüssige Milchgerinnungs-Aspartatprotease-Enzymzusammensetzung nach Anspruch 1, umfassend ein Konservierungsmittel, bevorzugt schwache organische Säuren wie etwa Formiat, Acetat, Lactat, Propionat, Malat, Benzoat, Sorbat oder Fumarat; oder Parabene wie etwa Alkylester von Parahydroxybenzoat; oder Glycerin oder Propandiol.

6. Flüssige Milchgerinnungs-Aspartatprotease-Enzymzusammensetzung nach Anspruch 1, wobei der pH-Wert der Zusammensetzung von 3 bis 7 beträgt, bevorzugt der pH-Wert von 4 bis 6,5 beträgt, mehr bevorzugt der pH-Wert von 5 bis 6 beträgt.

7. Flüssige Milchgerinnungs-Aspartatprotease-Enzymzusammensetzung nach Anspruch 1, wobei die Konzentration zugesetzter Polypeptide in Punkt II eine Konzentration von 0,5 % bis 4 % (Gew.-%) der Zusammensetzung ist; und

   wobei die flüssige Zusammensetzung ein Gesamtgewicht von 100 g bis 3000 kg aufweist; und
   (y): wobei die Summe der Mengen an Aspartatprotease-Enzym von Punkt I und Polypeptiden länger als 10 Aminosäuren von Punkt II, bestimmt durch SDS-PAGE, größer als 50 % (Gew.-%) der Gesamtmenge an Proteinen und/oder Polypeptiden länger als 10 Aminosäuren in der Zusammensetzung ist; und
   wobei die Polypeptide länger als 10 Aminosäuren mindestens ein Polypeptid ausgewählt aus der Gruppe von Polypeptiden bestehend aus Folgendem sind: alpha-Lactalbumin, beta-Lactoglobulin, Transferrin, Lactoperoxidase, Casein, alpha-S1-Casein, alpha-S2-Casein, beta-Casein, kappa-Casein, Ovalbumin und Gelatine.

8. Verfahren zum Herstellen eines Lebens- oder Futtermittelprodukts, umfassend Zusetzen einer wirksamen Menge einer Milchgerinnungs-Aspartatprotease-Enzymzusammensetzung nach einem der vorhergehenden Ansprüche zu dem oder den Lebens- oder der Futtermittelinhaltsstoff(en) und Durchführen weiterer Herstellungsschritte, um das Lebens- oder Futtermittelprodukt zu erhalten.

**Revendications**

1. Composition d'enzyme protéase aspartique liquide de coagulation du lait comprenant :

   I : une enzyme protéase aspartique de coagulation du lait à une concentration de 25 IMCU/g à 30 000 IMCU/g de la composition ;
   II : des polypeptides de plus de 10 acides aminés en une concentration de 0,01 % à 10 % (p/p) de la composition, dans laquelle les polypeptides de plus de 10 acides aminés étant au moins un polypeptide choisi dans le groupe constitué : d'alpha-lactalbumine, de bêta-lactoglobuline, de transferrine, de lactopéroxydase, de caséine, d'alpha-s1-caséine, d'alpha-s2-caséine, de bêta-caséine, de kappa-caséine, d'ovalbumine, de gélatine, de protéines de soja, de protéines de pois, de protéines de maïs, de protéines de pomme de terre, de protéines de chanvre, de protéines de riz, de protéines de spiruline, de protéines de blé, de protéines d'arachide, de protéines de tournesol, de protéines de graines de colza, de protéines sanguines et de protéines d'algues ; et

III : un sel en une concentration de 1 à 350 g/kg et dans laquelle le pH de la composition est de 2 à 8 ; et dans laquelle l'enzyme protéase aspartique de coagulation du lait est la chymosine (EC 3.4.23.4), dans laquelle l'enzyme protéase aspartique de coagulation du lait est la chymosine de *Camelius dromedarius* comprenant la séquence d'acides aminés polypeptidique représentée sur la figure 5 ici (appelée « Camel_chymosine ») ou une variante de *Camelius dromedarius* chymosine, dans laquelle la variante comprend une séquence polypeptidique qui a au moins 90 %, de préférence au moins 95 %, de manière davantage préférée au moins 99 %, d'identité de séquence avec la séquence d'acides aminés polypeptidique camel chymosine représentée sur la figure 5 ici, dans laquelle les unités internationales de coagulation du lait (IMCU) sont déterminées comme indiqué dans la description.

2. Composition d'enzyme protéase aspartique liquide de coagulation du lait selon la revendication 1, dans laquelle, par rapport au point II), les polypeptides de plus de 10 acides aminés sont à une concentration de 0,05 % à 8 % (p/p) de la composition ; de préférence à une concentration de 0,1 % à 7 % (p/p) de la composition ; de manière davantage préférée à une concentration de 0,25 % à 5 % (p/p) de la composition et encore plus préférablement à une concentration de 0,5 % à 4 % (p/p) de la composition, comme par exemple à une concentration de 1 % à 3 % (p/p) de la composition.

3. Composition d'enzyme protéase aspartique liquide de coagulation du lait selon la revendication 1, dans laquelle le sel au point (iii) est à une concentration de 10 à 300 g/kg, de préférence à une concentration de 25 à 250 g/kg.

4. Composition d'enzyme protéase aspartique liquide de coagulation du lait selon la revendication 1, dans laquelle le sel est un sel inorganique, de préférence dans laquelle le sel inorganique est choisi dans le groupe constitué de NaCl, de KCl, de $Na_2SO_4$, de $(NH_4)_2SO_4$, de $K_2HPO_4$, de $KH_2PO_4$, de $Na_2HPO_4$ ou de $NaH_2PO_4$ ou une combinaison de ceux-ci, de préférence le sel est NaCl.

5. Composition d'enzyme protéase aspartique liquide de coagulation du lait selon la revendication 1, comprenant un conservateur, de préférence des acides organiques faibles tels que le formiate, l'acétate, le lactate, le propionate, le malate, le benzoate, le sorbate ou le fumarate ; ou des parabènes tels que les esters alkyliques de parahydroxybenzoate ; ou du glycérol ou du propanediol.

6. Composition d'enzyme protéase aspartique liquide de coagulation du lait selon la revendication 1, dans laquelle le pH de la composition est de 3 à 7, de préférence le pH est de 4 à 6,5, de manière davantage préférée le pH est de 5 à 6.

7. Composition d'enzyme protéase aspartique liquide de coagulation du lait selon la revendication 1,

dans laquelle la concentration de polypeptides ajoutés au point II est à une concentration de 0,5 % à 4 % (p/p) de la composition ; et
dans laquelle la composition liquide a un poids total de 100 g à 3 000 kg ; et
(y) : dans laquelle la somme des quantités d'enzyme protéase aspartique du point I et des polypeptides de plus de 10 acides aminés du point II, déterminée par SDS-PAGE, est supérieure à 50 % (p/p) de la quantité totale de protéines et/ou de polypeptides de plus de 10 acides aminés dans la composition ; et
dans laquelle les polypeptides de plus de 10 acides aminés sont au moins un polypeptide choisi dans le groupe de polypeptides constitué : d'alpha-lactalbumine, de bêta-lactoglobuline, de transferrine, de lactoperoxydase, de caséine, d'alpha-s1-caséine, d'alpha-s2-caséine, de bêta-caséine, de kappa-caséine, d'ovalbumine et de gélatine.

8. Procédé de fabrication d'un aliment ou d'un produit alimentaire comprenant l'ajout d'une quantité efficace d'une composition d'enzyme protéase aspartique de coagulation du lait selon l'une quelconque des revendications précédentes à l'aliment ou à l'ingrédient/aux ingrédients alimentaire(s) et la réalisation d'autres étapes de fabrication pour obtenir l'aliment ou le produit alimentaire.

# Figure 1

CHY-MAX®

Hannilase®

# Figure 2

Inversion of enzymes
Sample:head space = 1:4 v/v

0.5% Casein hydrolysate

0.5% Alpha 20

# Figure 3

# Figure 4

```
Cow       GEVASVPLTNYLDSQYFGKIYLGTPPQEFTVLFDTGSSDFWVPSIYCKSNACKNHQRFDP
Buffalo   GEVASVPLTNYLDSQYFGKIYLGTPPQEFTVLFDTGSSDFWVPSIYCKSNACKNHQRFDP
Goat      GEVASVPLTNYLDSQYFGKIYLGTPPQEFTVLFDTGSSDFWVPSIYCKSNACKNHQRFDP
Sheep     GEVASVPLTNYLDSQYFGKIYLGTPPQEFTVLFDTGSSDFWVPSIYCKSNACKNHQRFDP
Camel     GKVAREPLTSYLDSQYFGKIYIGTPPQEFTVVFDTGSSDLWVPSIYCKSNVCKNHHRFDP
Pig       GEVASEPLTNYLDTQYFGKIYIGTPPQEFTVVFDTGSSELWVPSVYCKSDACQNHHRFNP
          *:**   ***.***:*******:************:******::****:****:.*:**:**:*

Cow       RKSSTFQNLGKPLSIHYGTGSMQGILGYDTVTVSNIVDIQQTVGLSTQEPGDVFTYAEFD
Buffalo   RKSSTFQNLGKPLSIRYGTGSMQGILGYDTVTVSNIVDIQQTVGLSTQEPGDVFTYAEFD
Goat      RKSSTFQNLGKPLSIRYGTGSMQGILGYDTVTVSNIVDTQQTVGLSTQEPGDVFTYAEFD
Sheep     RKSSTFQNLGKPLSIRYGTGSMQGILGYDTVTVSNIVDIQQTVGLSTQEPGDVFTYAEFD
Camel     RKSSTFRNLGKPLSIHYGTGSMEGFLGYDTVTVSNIVDPNQTVGLSTEQPGEVFTYSEFD
Pig       SKSSTFQNLDKPLSIQYGTGSIQGFLGYDTVMVAGIVDAHQTVGLSTQEPSDIFTYSEFD
          *****:**.*****:*****::*:****** *:.*** :*******::*.::***:***

Cow       GILGMAYPSLASEYSIPVFDNMMNRHLVAQDLFSVYMDRNGQESMLTLGAIDPSYYTGSL
Buffalo   GILGMAYPSLASEYSIPVFDNMMNRHLVAQDLFSVYMDRNGQESMLTLGAIDPSYYTGSL
Goat      GILGMAYPSLASEYSVPVFDNMMDRRLVAQDLFSVYMDRNGQGSMLTLGAIDPSYYTGSL
Sheep     GILGMAYPSLASEYSVPVFDNMMDRRLVAQDLFSVYMDRSGQGSMLTLGAIDPSYYTGSL
Camel     GILGLAYPSLASEYSVPVFDNMMDRHLVARDLFSVYMDRNGQGSMLTLGAIDPSYYTGSL
Pig       GILGLGYPELASEYTVPVFDNMMHRHLVAQDLFAVYMSRNDEGSMLTLGAIDPSYYTGSL
          ****:.**.*****::*******.*:***:***:***.*..: ****************

Cow       HWVPVTVQQYWQFTVDSVTISGVVVACEGGCQAILDTGTSKLVGPSSDILNIQQAIGATQ
Buffalo   HWVPVTVQQYWQFTVDSITISGVVVACEGGCQAILDTGTSKLVGPSSDILNIQQAIGATQ
Goat      HWVPVTLQKYWQFTVDSVTISGAVVACEGGCQAILDTGTSKLVGPSSDILNIQQAIGATQ
Sheep     HWVPVTLQKYWQFTVDSVTISGAVVACEGGCQAILDTGTSKLVGPSSDILNIQQAIGATQ
Camel     HWVPVTLQQYWQFTVDSVTINGVAVACVGGCQAILDTGTSVLFGPSSDILKIQMAIGATE
Pig       HWVPVTMQLYWQFTVDSVTINGVVVACNGGCQAILDTGTSMLAGPSSDILNIQMAIGATE
          ******:* ********:**.*..*** ************ * *******:** *****:

Cow       NQYGEFDIDCDNLSYMPTVVFEINGKMYPLTPSAYTSQDQGFCTSGFQSENHSQKWILGD
Buffalo   NQYGEFDIDCDNLSYMPTVVFEINGKMYPLTPSAYTSQDQGFCTSGFQSENRSQQWILGD
Goat      NQYGEFDIDCDSLSSMPTVVFEINGKMYPLTPYAYTSQEEGFCTSGFQGENHSHQWILGD
Sheep     NQYGEFDIDCDSLSSMPTVVFEINGKMYPLTPYAYTSQEEGFCTSGFQGENHSHQWILGD
Camel     NRYGEFDVNCGNLRSMPTVVFEINGRDYPLSPSAYTSKDQGFCTSGFQGDNNSELWILGD
Pig       SQYGEFDIDCGSLSSMPTVVFEISGRMYPLPPSAYTNQDQGFCTSGFQGDSKSQHWILGV
          .:*****::*..*  *******.*: ***.* ***.:::*******.:...*. ****

Cow       VFIREYYSVFDRANNLVGLAKAI
Buffalo   VFIREYYSVFDRANNLVGLAKAI
Goat      VFIREYYSVFDRANNLVGLAKAI
Sheep     VFIREYYSVFDRANNLVGLAKAI
Camel     VFIREYYSVFDRANNRVGLAKAI
Pig       VFIQEYYSVFDRANNRVGLAKAI
          ***:*********** ******
```

# Figure 5

```
Camel_chymosin    ----GKVAREPLTSYLDSQYFGKIYIGTPPQEFTVVFDTGSSDLWVPSIYCK-SNVCKNH
Cow_chymosin      ----GEVASVPLTNYLDSQYFGKIYLGTPPQEFTVLFDTGSSDFWVPSIYCK-SNACKNH
Cow_pepsin        --AATLVSEQPLQNYLDTEYFGTIGIGTPAQDFTVIFDTGSSNLWVPSIYCS-SEACTNH
Mucor             AAADGSVDTPGYYDFDLEEYAIPVSIGTPGQDFLLLFDTGSSDTWVPHKGCTKSEGCVGS
Endothia          ---STGSATTTPIDSLDDAYITPVQIGTPAQTLNLDFDTGSSDLWVFSSETT-ASEVDGQ
                            .       *    :  :*** * : : ******: **     . :.   .

Camel_chymosin    HRFDPRKSSTFRNLG-KPLSIHYGTG-SMEGFLGYDTVTVSNIVDPNQTVGLSTEQPGEV
Cow_chymosin      QRFDPRKSSTFQNLG-KPLSIHYGTG-SMQGILGYDTVTVSNIVDIQQTVGLSTQEPGDV
Cow_pepsin        NRFNPQDSSTYEATS-ETLSITYGTG-SMTGILGYDTVQVGGISDTNQIFGLSETEPGSF
Mucor             RFFDPSASSTFKATN-YNLNITYGTG-GANGLYFEDSIAIGDITVTKQILAYVDNVRGPT
Endothia          TIYTPSKSTTAKLLSGATWSISYGDGSSSSGDVYTDTVSVGGLTVTGQAVESAKKVSSSF
                   :  *   *:*   .  .    .* ** * . *     *::  :..:    * .      .

Camel_chymosin    FTYSE-----FDGILGLAYPS---LASEY---SVPVFDNMMDRHLVARDLFSVYMDRNGQ
Cow_chymosin      FTYAE-----FDGILGMAYPS---LASEY---SIPVFDNMMNRHLVAQDLFSVYMDRNGQ
Cow_pepsin        LYYAP-----FDGILGLAYPS---ISSSG---ATPVFDNIWDQGLVSQDLFSVYLSSNEE
Mucor             AEQSPNADIFLDGLFGAAYPDNTAMEAEYGSTYNTVHVNLYKQGLISSPLFSVYMNTNSG
Endothia          TEDST-----IDGLLGLAFST---LNTVSPTQQKTFFDNAKAS--LDSPVFTADLGYHAP
                       :        :**::* *:.     : :         ... *      :    :*:. :. :

Camel_chymosin    -GSMLTLGAIDPSYYTGSLHWVPVTLQQ----YWQFTVDSVTING-VAVACVGGCQAILD
Cow_chymosin      -ESMLTLGAIDPSYYTGSLHWVPVTVQQ----YWQFTVDSVTISG-VVVACEGGCQAILD
Cow_pepsin        SGSVVIFGDIDSSYYSGSLNWVPVSVEG----YWQITVDSITMNG-ESIACSDGCQAIVD
Mucor             -TGEVVFGGVNNTLLGGDIAYTDVMSRYGGYYFWDAPVTGITVDGSAAVRFSRPQAFTID
Endothia          --GTYNFGFIDTTAYTGSITYTAVSTKQG---FWEWTSTGYAVGS--GTFKSTSIDGIAD
                     . :*  :: :    *.: :. * .    :*: .  . ::..         *

Camel_chymosin    TGTSVLFGPSSDILKIQMAIG--ATENRYGEFDVNCGNLRSMPTVVFEINGRDYPLSPSA
Cow_chymosin      TGTSKLVGPSSDILNIQQAIG--ATQNQYGEFDIDCDNLSYMPTVVFEINGKMYPLTPSA
Cow_pepsin        TGTSLLAGPTTAISNIQSYIG--ASEDSSGEVVISCSSIDSLPDIVFTINGVQYPVPPSA
Mucor             TGTNFFIMPSSAASKIVKAALPDATETQQGWVVPCASYQNSKSTISIVMQKSGSSSDTIE
Endothia          TGTTLLYLPATVVSAYWAQVSGAKSSSSVGGYVFPCS--ATLPSFTFGVGSARIVIPGDY
                  ***. :  *::         :.  *    ..      . . : :

Camel_chymosin    YTS-KDQGFCTSGFQ---------GDNNSELWILGDVFIREYYSVFDRANN-RVGLAKAI
Cow_chymosin      YTS-QDQGFCTSGFQ---------SENHSQKWILGDVFIREYYSVFDRANN-LVGLAKAI
Cow_pepsin        YIL-QSNGICSSGFEG-----MDISTSSGDLWILGDVFIRQYFTVFDRGNN-QIGLAPVA
Mucor             ISV-PVSKMLLPVDQSNETCMFIILPDGGNQYIVGNLFLRFFVNVYDFGNN-RIGFAPLA
Endothia          IDFGPISTGSSSCFGG------IQSSAGIGINIFGDVALKAAFVVFNGATTPTLGFASK-
                    .      .          *.*:: ::     *:: ...   :*:*

Camel_chymosin    ------
Cow_chymosin      ------
Cow_pepsin        ------
Mucor             SAYENE
Endothia          ------
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2333056 A1
- WO 2012127005 A1
- DE 1492060 A1
- WO 0236752 A2

**Non-patent literature cited in the description**

- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277